# EUROPEAN PATENT APPLICATION

(11) **EP 4 250 300 A2**
(43) Date of publication of application: **27.09.2023**
(21) Application number: 23164752.0
(22) Date of filing: 07.02.2020
(51) Int. Cl.: G16B 30/10

(54) **BIOLOGICAL SEQUENCE INFORMATION HANDLING**

(30) Priority: 07.02.2019 BE 201905077; 07.02.2019 EP 19156085; 08.08.2019 EP 19190899
(62) Divisional of application: 20704821.6
(71) Applicant: BioKey BV, 3590 Diepenbeek (BE)
(72) Inventor: VAN HYFTE, Dirk, 3930 Hamont (BE); VAN HYFTE, Arnout, 3001 Heverlee (BE); BRANDS, Ingrid, 3930 Hamont (BE); VAN HYFTE, Ewald, 3930 Hamont (BE)
(74) Representative: Winger

(57) **Abstract**

In a first aspect, the present invention relates to a repository of fingerprint data strings for a biological sequence database, each fingerprint data string representing a characteristic biological subsequence made up of sequence units, each characteristic biological subsequence having in the biological sequence database a combinatory number which is lower than the total number of different sequence units available thereto, the combinatory number of a biological subsequence being defined as the number of different sequence units that appear in the biological sequence database as a consecutive sequence unit of the biological subsequence.

SEQUENCE LISTING

<110> Biostrand BVBA
      BioKey BVBA

<120> BIOLOGICAL SEQUENCE INFORMATION HANDLING

<130> 20022VTr00WO/dw/ac/av

<140> EPPCTNYK
<141> 2020-02-07

<150> EP19190899.5
<151> 2019-08-08
<150> BE2019/5077
<151> 2019-02-07
<150> EP19156085.3
<151> 2019-02-07

<160> 10

<170> BiSSAP 1.3.6

<210> 1
<211> 7
<212> PRT
<213> Unknown

<220>
<223> Unknown

<400> 1
Met Cys Met His Asn Gln Ala
1               5

<210> 2
<211> 6
<212> PRT
<213> Unknown

<220>
<223> Unknown

<400> 2
Met Cys Met His Asn Gln
1               5

<210> 3
<211> 25
<212> PRT
<213> Unknown

<220>
<223> Unknown

<400> 3
Ala Val Phe Pro Ser Ile Val Gly Arg Pro Arg His Gln Gly Val Met
1               5                   10                  15
Val Gly Met Gly Gln Lys Asp Ser Tyr
            20                  25

<210> 4
<211> 11
<212> PRT
<213> Unknown

<220>
<223> Unknown

<400> 4
Ile Val Gly Arg Pro Arg His Gln Gly Val Met
1               5                   10

<210> 5
<211> 11
<212> PRT
<213> Unknown

<220>
<223> Unknown

<400> 5
Phe Ala Gly Asp Asp Ala Pro Arg Ala Val Phe
1               5                   10

<210> 6
<211> 11
<212> PRT
<213> Unknown

<220>
<223> Unknown

<400> 6
Ile Val Gly Arg Pro Arg His Gln Gly Val Met
1               5                   10

<210> 7
<211> 11
<212> PRT
<213> Unknown

<220>
<223> Unknown

<400> 7
Ile Ile Thr Asn Trp Asp Asp Met Glu Lys Ile
1               5                   10

<210> 8
<211> 11
<212> PRT
<213> Unknown

<220>
<223> Unknown

<400> 8

Ile Met Phe Glu Thr Phe Asn Val Pro Ala Met
1               5                   10

<210> 9
<211> 11
<212> PRT
<213> Unknown

<220>
<223> Unknown

<400> 9
Ile Lys Glu Lys Leu Cys Tyr Val Ala Leu Asp
1               5                   10

<210> 10
<211> 11
<212> PRT
<213> Unknown

<220>
<223> Unknown

<400> 10
Ile Gly Met Glu Ser Ala Gly Ile His Glu Thr
1               5                   10

## Description

### Technical field of the invention

The present invention relates to the handling of biological sequence information, including for example processing, storing and comparing said biological sequence information.

### Background of the invention

Biological sequencing has evolved at a blinding speed in the last decades, enabling along the way the human genome project which achieved a complete sequencing of the human genome already more than 15 years ago. To fuel this evolution, ample technical progress has been required, spanning from advances in sample preparation and sequencing methods to data acquisition, processing and analysis. Concurrently, new scientific fields have spawned and developed, including genomics, proteomics and bioinformatics.

Fuelled by the postgenomic era's emphasis on data acquisition, this evolution has resulted in the accumulation of enormous amounts of sequence data. However, the ability to organize, analyse and interpret this sequence, to extract therefrom biologically relevant information, has been trailing behind. This problem is further compounded by the magnitude of new sequence information which is still generated on a daily basis. Muir et al. observed that this is sparking a paradigm shift and have commented on the resulting changing cost structure for sequencing and other associated hurdles (MUIR, Paul, et al. The real cost of sequencing: scaling computation to keep pace with data generation. Genome biology, 2016, 17.1: 53.).

Accessing, analysing or employing sequence information in a meaningful way generally requires the need for a form of sequence alignment and similarity search. An abundant amount of computer software is commercially available to perform such alignments and sequence similarity searches, e.g. BLAST, PSI-BLAST; SSEARCH, FASTA, HMMER3. Nevertheless, the known algorithms lack the speed or practical ability to process the vast amount of already existing data. Hardware optimizations have also been attempted, such as disclosed in US2006020397A1, but have not brought the necessary breakthrough. At the core of this struggle is that the problem which is being addressed is of the NP-hard or NP-complete nature (NP = non-deterministic polynomial-time); as such, the required resources scale exponentially as the difficulty of the task increases (e.g. with increasing sequence length or with increasing number of sequences to be compared).

Genome graphs are used as a reference in the processing, storing or comparing sequences, such sequences being typically reconstructed from single reads, which typically are shorter sequences of DNA or RNA. A linear reference thereby is a representation of one single genome. For a complete representation, multiple genomes need to be combined in order to find all variations a specie can have.

Multiple problems arise in correctly constructing a Pangenome graph. First, even the best assembled reference genomes contain gaps and errors. Secondly, one cannot find a suitable graph representation to enclose al necessary information to counter problems that later arise when the process of graph mapping is to be performed. Nor a De Bruijn graph, directed graph or bidirected graph can accurately represent strands. Third it seems possible to create a reference cohort using current techniques, but the constructed cohort is essentially not usable in practice due to the lack of structural coordinates.

Further, graphs lack operational site definitions. Because of the logarithmic complexity, repeating areas are even harder to represent using the known k-mer based technology. Concluding, it is nearly impossible to construct a cohort of variations in a graph structure for 1 specie, let alone impossible to construct one for all biological species, due to the impossibility to keep all necessary data using state of the art techniques.

Structural variants play an important role in the development of cancer and other diseases and are less well studied than single nucleotide variations, in part due to the lack of reliable identification from read data. When k-mer technology is used, the detection window for variations is per definition smaller than the total length of the k-mer. Using algorithms for overcoming the k-mer window problem, one cannot effectively identify structural variances. High coverages are needed to find evidence for just one structural variation. Therefore, the usage of k-mers needs a large pool before real variations can effectively be identified from noise and read errors. A lot of k-mers leads to a hard computational problem due to the lack of dynamic algorithms to align k-mers. This illustrates the need for heuristics or parameterization to shrink the search space. The latter nevertheless results in inevitable error accumulation which shows that k-mers are not effective unified spatial patterns. At present this is only solved in a syntactic way which is strictly mono-dimensional.

Due to the NP-hard nature of the mapping and assembly process, greedy algorithms typically are used to solve these problems, whereby from a certain input an expansion matrix is used to compute relevant results.

Dynamic programming has been used, but the problems associated therewith is that the source data (parameters like position, read ID, etc.) are lost and backtracking is not possible anymore.

All of the above problems make efficient and accurate graph collapsing near impossible. This results in the impossibility to provide the necessary accuracy or positional data required to construct a usable pangenomic graph. In addition usage of k-mers lack specificity to differentiate multi-dimensional parameters in genetic information. This further ads to the inefficient construction of current genomic graphs, shown by the inability to call structural variance, biases or effectively enclose high repetitive regions.

There is thus still a need in the art for ways to efficiently tap into sequence information, allowing to extract and use the relevant information therein to address a particular problem.

### Summary of the invention

It is an object of the present invention to provide a good way to handle biological sequence information. This objective is accomplished by methods, devices and data structures according to the present invention.

In a first aspect, the present invention relates to a repository of fingerprint data strings for a biological sequence database, each fingerprint data string representing a characteristic biological subsequence made up of sequence units, each characteristic biological subsequence having in the biological sequence database a combinatory number which is lower than the total number of different sequence units available thereto, the combinatory number of a biological subsequence being defined as the number of different sequence units that appear in the biological sequence database as a consecutive sequence unit of the biological subsequence.

It is an advantage of embodiments of the present invention that a repository of fingerprint data strings corresponding to characteristic biological subsequences can be provided. It is a further advantage of embodiments of the present invention that the biological subsequences need not be of a single length, as is the case for e.g. k-mers.

It is an advantage of embodiments of the present invention that further data, e.g. metadata, can be included in the repository, such as data on the sequence unit(s) which may be consecutive to (i.e. succeeding directly after or preceeding directly before) a characteristic biological subsequence, data on the secondary/tertiary/quaternary structure of a characteristic biological subsequence (e.g. when said characteristic biological subsequence is present in a biopolymer), data on a relationship between fingerprints (e.g. data related to a relationship between the characteristic biological subsequence and one or more further characteristic biological subsequences), etc.

In a second aspect, the present invention relates to a computer-implemented method for building and/or updating a repository of fingerprint data strings as defined in any embodiment of the first aspect, comprising: (a) identifying a characteristic biological subsequence in a biological sequence database, the characteristic biological subsequence having a combinatory number which is lower than the total number of different sequence units available thereto, the combinatory number of a biological subsequence being defined as the number of different sequence units that appear in the biological sequence database as a consecutive sequence unit of the biological subsequence; (b) optionally, translating the identified characteristic biological subsequence to one or more further characteristic biological subsequences; and (c) populating said repository with one or more fingerprint data strings representing the identified characteristic biological subsequence and/or the one or more further characteristic biological subsequences.

In a third aspect, the present invention relates to a computer-implemented method for processing a biological sequence, comprising: (a) retrieving one or more fingerprint data strings from the repository of fingerprint data strings as defined in any embodiment of the first aspect, (b) searching the biological sequence for occurrences of the characteristic biological subsequences represented by the one or more fingerprint data strings, and (c) constructing a processed biological sequence comprising for each occurrence in step b a fingerprint marker associated with the fingerprint data string which represents the occurring characteristic biological subsequence.

It is an advantage of embodiments of the present invention that systems and methods are obtained, providing reduced complexity.

It is an advantage of embodiments of the present invention that systems and methods are obtained that are deterministic, i.e. lead to a given solution.

It is an advantage of embodiments of the present invention that a biological sequence can be relatively easily and efficiently processed. It is a further advantage of embodiments of the present invention that a biological sequence can be analysed in a lexical or even a semantic fashion.

It is an advantage of embodiments of the present invention that the processed biological sequence can be constructed by replacing therein the identified characteristic biological subsequences by markers associated with the corresponding fingerprint data strings.

It is an advantage of embodiments of the present invention that the portions of the biological sequence which do not correspond to one of the characteristic biological subsequences can be handled in a variety of ways. It is a further advantage of some embodiments that the biological sequence can be processed in a completely lossless way (i.e. no information is lost by processing). It is a further advantage of alternative embodiments of the present invention that the biological sequence can be processed in a way that the more important information is distilled in a more condensed format.

It is an advantage of embodiments of the present invention that the processed biological sequences may be compressed so that they take up less storage space than their unprocessed counterparts.

It is an advantage of embodiments of the present invention that matching portions of the biological sequence to the characteristic biological subsequences is not solely limited to the primary structure, but can also take into account the secondary/tertiary/quaternary structure.

It is an advantage of embodiments of the present invention that a secondary/tertiary/quaternary structure of a biological subsequence can be at least partially elucidated based on the known secondary/tertiary/quaternary structure of characteristic biological subsequences contained therein. It is a further advantage of embodiments of the present invention that biological sequence design (e.g. protein) design can be assisted or facilitated.

It is an advantage of embodiments of the present invention that lossless compression is obtained. More particularly, without information loss, due to the use of the HYFTs^{™}, the required computational capacity is far more limited, resulting in a workable solution.

It is an advantage of embodiments of the present invention that by using HYFTs^{™} which inherently include directionality, a suitable graph representation is provided that encloses all necessary information to counter problems that arise when processing for graph mapping is required.

It is an advantage of embodiments of the present invention that the system and methods allow for a large flexibility and/or scalability.

It is an advantage of embodiments of the present invention that the analysis is not a NP-hard problem anymore, and therefore has far less computational requirements compared to existing methods and systems providing similar results. The latter can be obtained since there is no need for expansion matrix-based steps or parametrisation steps.

In a fourth aspect, the present invention relates to a processed biological sequence, obtainable by the computer-implemented method according to any embodiment of the third aspect.

In a fifth aspect, the present invention relates to a computer-implemented method for building and/or updating a repository of processed biological sequences, comprising populating said repository with processed biological sequences as defined in any embodiment of the fourth aspect.

It is an advantage of embodiments of the present invention that a repository of processed biological sequences can be constructed and stored.

It is an advantage of embodiments of the present invention that the repository is updatable without having to recalculate the full repository.

In a sixth aspect, the present invention relates to a repository of processed biological sequences, obtainable by the computer-implemented method according to any embodiment of the fifth aspect.

It is an advantage of embodiments of the present invention that the repository of processed biological sequences can be quickly searched and navigated. It is a further advantage of embodiments of the present invention that the storage size of the repository may be relatively small, compared to the known databases, by populating it with compressed processed biological sequences.

It is an advantage of embodiments of the present invention that-once build-the repository can be stored, maintained and updated as desired; i.e. it does not need to be recalculated for each use.

In a seventh aspect, the present invention relates to a computer-implemented method for comparing a first biological sequence to a second biological sequence, comprising: (a) processing the first biological sequence by the computer-implemented method according to any embodiment of the third aspect to obtain a first processed biological sequence, or retrieving the first processed biological sequence from a repository of processed biological sequences as defined in any embodiment of the sixth aspect, (b) processing the second biological sequence by the computer-implemented method according to any embodiment of the third aspect to obtain a second processed biological sequence, or retrieving the second processed biological sequence from a repository of processed biological sequences as defined in any embodiment of the sixth aspect, and (c) comparing at least the fingerprint markers in the first processed biological sequence with the fingerprint markers in the second processed biological sequence.

It is an advantage of embodiments of the present invention that the comparison of biological sequences can be changed from an NP-complete or NP-hard problem to a polynomial-time problem. It is a further advantage of embodiments of the present invention that comparison can be performed in a greatly reduced time and scales well with increasing complexity (e.g. increasing length of or number of biological sequences). It is yet a further advantage of embodiments of the present invention that the required computational power and storage space can be reduced.

It is an advantage of embodiments of the present invention that a degree of similarity can be calculated between biological sequences. It is a further advantage of embodiments of the present invention that a plurality of biological sequences can be ranked based on their degree of similarity.

It is an advantage of embodiments of the present invention that a sequence similarity search can be quickly and easily performed (e.g. in polynomial time). It is a further advantage of embodiments of the present invention that compared biological sequences can be easily and quickly aligned (e.g. in polynomial time). It is yet a further advantage of embodiments of the present invention that biopolymer sequences (e.g. of biopolymer fragments) can after alignment be easily and quickly merged (e.g. to reconstruct the original biopolymer sequence, such as in a sequence assembly).

It is an advantage of embodiments of the present invention that also a plurality of sequences can be easily and quickly compared, aligned and/or merged. It is a further advantage of embodiments of the present invention that there is no accumulation of errors during the alignment, as is the case in currently known methods (e.g. based on progressive alignment).

In an eighth aspect, the present invention relates to a storage device comprising a repository of fingerprint data strings according to any embodiment of the first aspect and/or a repository of processed biological sequences according to any embodiment of the sixth aspect.

In a ninth aspect, the present invention relates to a data processing system adapted to carry out the computer-implemented method according to any embodiment of the second, third, fifth or seventh aspect.

It is an advantage of embodiments of the present invention that the methods may be implemented by a variety of systems and devices, such as computer-based systems or a sequencer, depending on the application. It is a further advantage of embodiments of the present invention that the methods can be implemented by a computer-based system, including a cloud-based system.

In a tenth aspect, the present invention relates to a computer program comprising instructions which, when the program is executed by a computer, cause the computer to carry out a computer-implemented method according to any embodiment of the second, third, fifth or seventh aspect.

In an eleventh aspect, the present invention relates to a computer-readable medium comprising instructions which, when executed by a computer, cause the computer to carry out a computer-implemented method according to any embodiment of the second, third, fifth or seventh aspect.

In a twelfth aspect, the present invention relates to a use of a repository of fingerprint data strings as defined in any embodiment the first aspect, for one or more selected from: processing a biological sequence, building a repository of processed biological sequences, comparing a first biological sequence to a second biological sequence, aligning a first biological sequence to a second biological sequence, performing a multiple sequence alignment, performing a sequence similarity search and performing a variant calling.

In a thirteenth aspect, the present invention relates to a use of a processed biological sequence as defined in any embodiment of the fourth aspect or a repository of processed biological sequences as defined in any embodiment as defined in any embodiment of the sixth aspect, for one or more selected from: comparing a first biological sequence to a second biological sequence, aligning a first biological sequence to a second biological sequence, performing a multiple sequence alignment, performing a sequence similarity search and performing a variant calling.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG 1 and FIG 2 are graphs showing expected progress enabled by embodiments of the present invention.
FIG 3 to FIG 5 are diagrams depicting systems in accordance with embodiments of the present invention.
FIG 6 to FIG 10 are charts showing various indicators with respect to the analysis of the processed Protein Data Bank (PDB) in accordance with embodiments of the present invention.
FIG 11 is a chart plotting against one another the number of HYFT^{™} matches found in the PDB database using two different matching strategies.
FIG 12 and FIG 15 are graphs comparing the total length of search results using, on the one hand, a prior art method (dotted line) and, on the other hand, a method in accordance with exemplary embodiments of the present invention (solid line).
FIG 13 and FIG 16 are graphs comparing the Levenshtein distance of search results using, on the one hand, a prior art method (dotted line) and, on the other hand, a method in accordance with exemplary embodiments of the present invention (solid line).
FIG 14 and FIG 17 are graphs comparing the longest common substring of search results using, on the one hand, a prior art method (dotted line) and, on the other hand, a method in accordance with exemplary embodiments of the present invention (solid line).

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms before, after, and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, a biological sequence is a sequence of a biopolymer defining at least the biopolymer's primary structure. The biopolymer can for example be a deoxyribonucleic acid (DNA), ribonucleic acid (RNA) or a protein. The biopolymer is typically a polymer of biomonomers (e.g. nucleotides or amino acids), but may in some instances further include one or more synthetic monomers.

As used herein, a 'sequence unit' in a biological sequence is an amino acid when the biological sequence relates to a protein and is a codon when the biological sequence relates to DNA or RNA.

As used herein, a biological subsequence is a portion of a biological sequence, smaller than the full biological sequence. The biological subsequence may, for example, have a total length of 100 sequence units or less, preferably 50 or less, yet more preferably 20 or less.

As used herein, a distinction is made between a 'characteristic biological subsequence' (or '(HYFT^{™}) fingerprint'), a '(HYFT^{™}) fingerprint data string' and a '(HYFT^{™}) fingerprint marker'. The first is a subsequence with particular characteristics as explained in more detail below. The second is data representation of such a HYFT^{™} fingerprint-optionally in combination with additional data (cf. infra)-, which may for example be stored in a corresponding repository. In some embodiments, one HYFT^{™} fingerprint data string may simultaneously represent multiple equivalent HYFT^{™} fingerprints (e.g. equivalent through coding for the same outcome, such as in the case of multiple codons which code for the same amino acid, or equivalent through translation; cf. infra). The third is a pointer to a HYFT^{™} fingerprint, such as a memory address where the HYFT^{™} fingerprint can be located or a reference allowing to find the HYFT^{™} fingerprint in a repository of fingerprint data strings. Nevertheless, given their close relationship, where a strict distinction between these three terms does not need to be drawn or where the meaning is clear in the context, these may herein simply be referred to as 'HYFTs^{™}'.

As used herein, a distinction is made between a 'biological sequence' and a 'processed biological sequence'. The former being a biological sequence as is widely known in the art, while latter is reconstructed/rewritten biological sequence which comprises fingerprint markers associated with the HYFT^{™} fingerprints of the present invention.

It will be clear that neither HYFT^{™} fingerprint data strings, nor processed biological sequences, nor the repositories storing these can be considered cognitive data and they are not targeted to (human) users. Instead, they are intended to be used as functional data in various computer-implemented methods by a computer (or similar technical system) and are structured to that effect. For example, the repositories may be structures as a relational database (e.g. based on SQL) or a NoSQL database (e.g. a document-oriented database, such as an XML database). Likewise, the HYFT^{™} fingerprint data strings and/or processed biological sequences may be structured as suitable entries for such databases.

As used herein, some concepts will be illustrated with examples relating to proteins and it will be assumed that the possible monomeric sequence units are the 20 canonical (or 'standard') amino acids. However, it is clear that this is merely to simplify the illustration and that similar embodiments can likewise be formulated with an extended number of amino acids (e.g. adding non-canonical amino acids or even synthetic compounds), or relating to DNA or RNA. In the case of DNA or RNA, a link between the DNA or RNA and proteins can be easily made through the correspondence between codons and amino acids.

As used herein, 'secondary/tertiary/quaternary' refers to 'secondary and/or tertiary and/or quaternary'.

It was surprisingly realized within the present invention that, where it was previously assumed that the primary structure of a biological sequence consists of an essentially independent selection of sequence units, so that there are in principle e.g. mⁿ biological sequences of length n based on m possible sequence units (e.g. 20ⁿ based on 20 canonical amino acids), this is in fact not observed in nature. Indeed, it was discovered that from a certain length onwards, not every theoretical combination is seen. To give but one example: the protein subsequence 'MCMHNQA' is not found in any protein in the public databases. It has been contemplated that this is not a mere hiatus in the databases, but that this absence has a physical and/or chemical origin. Without being bound by theory, to name but one possible effect, the steric hindrance of the neighbouring amino acids (e.g. 'MCMHNQ' in the above example) may prohibit one or more other amino acids (e.g. 'A' in the above example) from binding thereto. As such, once an absent subsequence has been identified, computational studies can be used to validate whether this subsequence could potentially occur or whether its existence is physically impossible (or improbable, e.g. because it's chemically unstable). The 'certain length' referred to above depends on the data set that is being considered, but e.g. corresponds to about 5 or 6 amino acids for the publicly available protein sequence databases (which substantially reflect the total diversity seen in nature). For a more limited set (e.g. a set filtered based on a particular criterion or formulated for a specific biological sequence database, such as for a specific domain), less than the theoretical maximum of mⁿ combinations is already found for a length of about 4 or 5.

Simultaneously, because the subsequence 'MCMHNQA' does not exist, the subsequence 'MCMHNQ' is not merely a random combination of 5 amino acids but gains additional significance; such subsequences will be further referred to as 'characteristic biological subsequences' or '(HYFT^{™}) fingerprints'. Because of the added significance or meaning of these HYFT^{™} fingerprints, it can be considered that the present invention handles biological sequence information in a more semantic fashion. In general, a characteristic subsequence is characterized by having for the sequence unit directly following (or preceding) it less possible options (i.e. a lower combinatory number) than the maximum number of sequence units (i.e. the total number of different sequence units available therefor; e.g. less than the 20 canonical amino acids); in other words, at least one of the sequence units cannot follow (or precede) it. However, it is possible to select a stricter definition: e.g. only those subsequences which have 15 sequence units or fewer which can possibly follow it, or 10 or fewer, 5 or fewer, 3, 2 or even 1. Furthermore, it can be chosen to consider each such subsequence as a HYFT^{™} fingerprint, or to consider only those subsequences as HYFT^{™} fingerprints which do not already comprise another HYFT^{™} fingerprint (i.e. which are non-redundant). For example: taking 'MCMHNQ' as a HYFT^{™} fingerprint, there will be longer subsequences which comprise 'MCMHNQ' and which also have less than the theoretical number of sequence units which can follow (or precede) it; in that case, there is the option to consider both the longer subsequences and 'MCMHNQ' as HYFT^{™} fingerprints, or to consider only 'MCMHNQ' as a HYFT^{™} fingerprint. The latter approach may typically be preferred in orderto keep the size of the repository of HYFT^{™} data strings in check, while speeding up the methods related thereto. Indeed, searching a biological sequence for matches with a string typically becomes more resource intensive and slower as the length of the string increases. Moreover, as the size of the repository of HYFT^{™} data strings increases, searching and retrieving a particular HYFT^{™} data string typically takes longer. In this non-redundant approach, longer subsequences with limited combinatory possibilities can still be identified, but then as patterns of HYFTs^{™} (with or without interdistances). As such, the advantages offered by this approach do not necessarily entail a corresponding loss of information. The aforementioned notwithstanding, note that the former approach is nevertheless also possible and doing so remains advantageous over the prior art.

It was then surprisingly found that a limited set of characteristic biological subsequences can be identified. Furthermore, it was observed that these characteristic biological subsequences strike a balance between, on the one hand, being sufficiently specific so that not every characteristic biological subsequence is found in every biological sequence and, on the other hand, being common enough that the known biological sequences typically comprise at least one of these HYFT^{™} fingerprints.

Out of the account provided above, a protocol for identifying HYFT^{™} fingerprints and building a corresponding repository of HYFT^{™} data strings (or 'HYFT^{™} repository') can be formulated. Indeed, since the objective is to identify those subsequences which have limited combinatory possibilities in a biological sequence database, it suffices to mine said biological sequence database for subsequences which do not appear therein. Once such a non-occurring subsequence (e.g. 'MCMHNQA') is identified, a subsequence which is one sequence unit shorter (e.g. 'MCMHNQ') corresponds to a HYFT^{™} fingerprint (provided that shorter subsequence does appear). Once identified, additional data on the HYFT^{™} fingerprint can then be derived. For example, the combinatory number can obtained by searching the biological sequence database for the combinations of the identified HYFT^{™} fingerprint with the other sequence units (e.g. replacing 'A' in 'MCMHNQA' each time with one of the other possible amino acids) and counting the number of combinations which are found to appear. Optionally, the combinations which are not found may also be stored separately; these may for example be used for error detection. Moreover, since the correspondence between DNA, RNA and proteins is typically known through the applicable codon tables, once a HYFT^{™} fingerprint of a particular type is identified (e.g. a protein HYFT^{™}), it can be translated to a corresponding HYFT^{™} fingerprint of a different type (e.g. a DNA and/or RNA HYFT^{™}). By repeating the above process and storing at least the identified HYFTs^{™} in a suitable format-optionally together with any additional data and translated HYFTs^{™}-a repository of HYFT^{™} fingerprint data strings can be build up. Alternatively or complementary thereto, at least some HYFT^{™} fingerprints could be found by experimental or computational methods, e.g. through synthesizing or modelling various subsequences and subsequently identifying those subsequences which cannot—or are too unlikely-to appear in the context of the biological sequence database under consideration.

In the above, the biological sequence database may be a publicly available database, such as the Protein Data Bank (PDB), or a proprietary database. In embodiments, the biological sequence database may be a combination of a plurality of individual database. For example, a repository of HYFT^{™} fingerprint data strings can be formulated from a biological sequence database combining as many (trustworthy) biological sequence databases as can be accessed, thereby seeking to come to a general repository of HYFT^{™} fingerprint data strings that is substantially representative for all biological sequences found in nature. Conversely, in a particular domain, it may prove fruitful to build a specific repository of HYFT^{™} fingerprint data strings based on a biological sequence database which is representative for that particular domain. Such a specific repository could in embodiments contain HYFTs^{™} which are absent in the general repository, because they do appear in nature but not within this particular domain. Likewise, a repository of HYFT^{™} fingerprint data strings could be built for synthetic sequences, with its own specific contents.

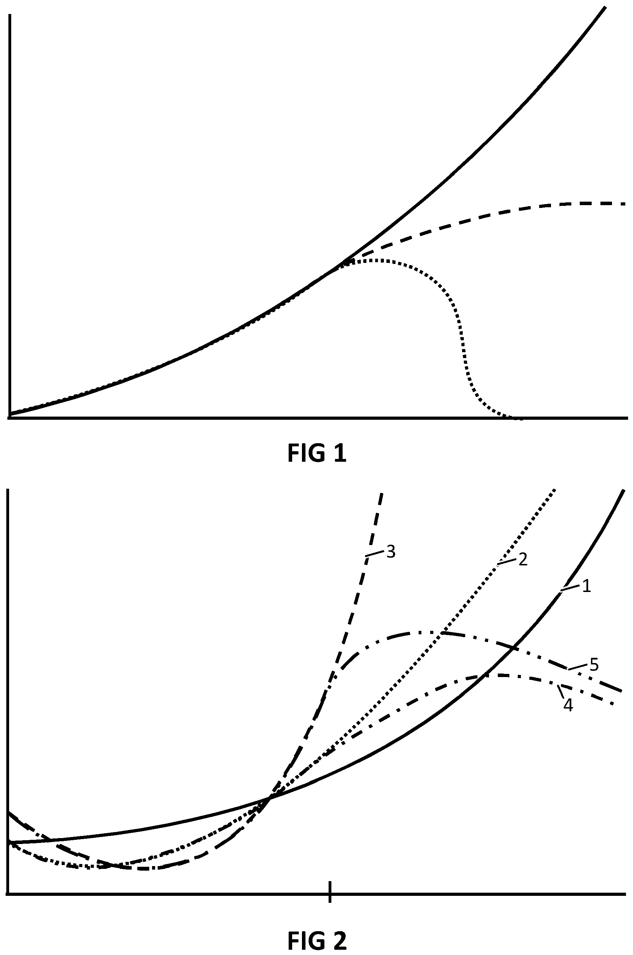

Based on the above discovery, new approaches to handling biological sequence information, in all its different but interrelated stages, can be formulated. These approaches can be considered as being akin to a more lexical analysis of the sequences. The result is schematically depicted in FIG 1, which shows the complexity scaling of the biological sequence information with an increasing number of sequence units (n). This complexity may be the total number of possible combinations of sequence units, but that in turn also relates to the computational effort (e.g. time and memory) needed for handling it (e.g. for performing a similarity search). The solid curve depicts the number of theoretical combinations assuming all sequence units are selected independently, scaling as mⁿ, which also corresponds to the scaling of the currently known algorithms. The dashed curve depicts the number of actual combinations found in nature (as observed within the present invention), where the curve departs from mⁿ at around 5 or 6 sequence units and asymptotically flattens off for high n. The dotted line shows the number of sequences which correspond for the first time to a characteristic sequence for which the number of sequence units which can follow it is equal to 1; here 'for the first time' means that longer sequences are never counted if they comprise an already counted HYFT^{™} fingerprint. Thus, the latter corresponds to the number of HYFT^{™} fingerprints of length n (as observed within the present invention), when the definition thereof is selected as a subsequence which has only 1 sequence unit which can possibly follow it and which does not already comprise another (shorter) HYFT^{™} fingerprint (cf. supra).

FIG 2 depicts the predicted benefits of the present invention in time, where the mark on the bottom axis depicts the present day. Curve 1 shows Moore's law as a reference. Curve 2 shows the total amount of acquired sequencing data. Curve 3 shows the total cost of processing and maintaining said sequencing data. By handling biological sequence information as proposed in the present invention, the total required storage for sequencing data and the total cost of data processing and maintenance are expected to drop as depicted in curves 4 and 5 respectively.

Note that, while a repository of HYFT^{™} fingerprint data strings is typically build with respect to a particular biological sequence database (or a combination thereof), this does not mean that the HYFT^{™} fingerprint data strings is only suitable for handling biological sequences in that particular biological sequence database. Indeed, a general repository of HYFT^{™} fingerprint data strings could for example be used for processing more specific biological sequences. In other cases, a specific repository of HYFT^{™} fingerprint data strings could be used in the context of a biological sequence falling outside of database used to formulate the repository. In both instances, advantageous results can still be obtained. In any case, one can always determine by trial-and-error whether an existing repository of HYFT^{™} fingerprint data strings can be used for a particular application or whether better results are obtained with a repository of HYFT^{™} fingerprint data strings dedicated thereto. Likewise, the repository of HYFT^{™} fingerprint data strings does not strictly need to encompass all HYFT^{™} fingerprints that could be found out in the biological sequence database. Indeed, a partial repository already yields beneficial results. Such a partial repository could for example be one related to HYFT^{™} fingerprints of selected lengths (i.e. as opposed to HYFT^{™} fingerprints of any length).

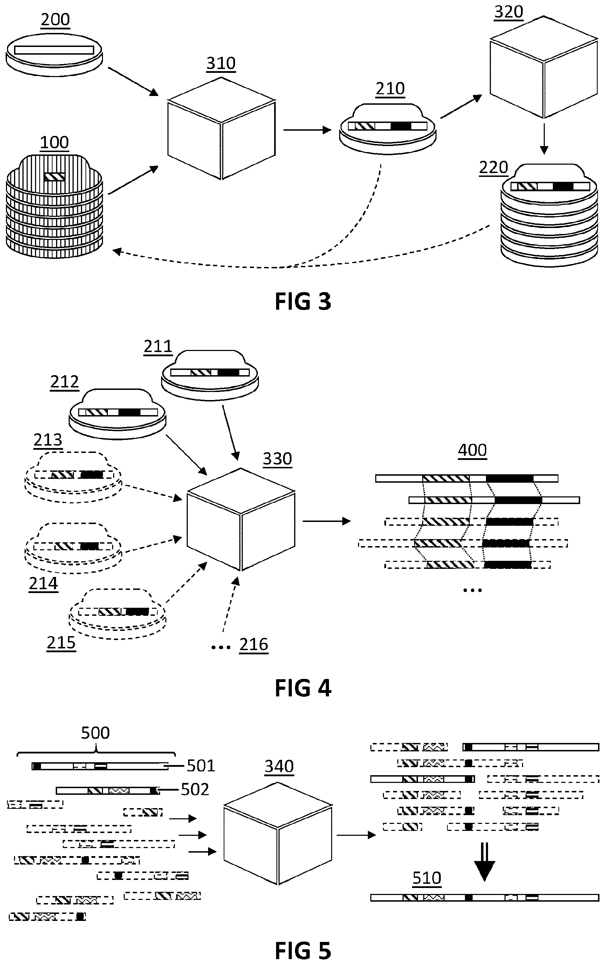

In a first aspect, the present invention relates to a repository of fingerprint data strings for a biological sequence database, each fingerprint data string representing a characteristic biological subsequence made up of sequence units, each characteristic biological subsequence having in the biological sequence database a combinatory number which is lower than the total number of different sequence units available thereto, the combinatory number of a biological subsequence being defined as the number of different sequence units that appear in the biological sequence database as a consecutive sequence unit of the biological subsequence. A repository (e.g. database) of fingerprint data strings 100 is schematically depicted in FIG 3, which will be discussed in more detail below.

In embodiments, the repository may comprise at least a first fingerprint data string representing a first characteristic biological subsequence of a first length and a second fingerprint data string representing a second characteristic biological subsequence of a second length, wherein the first and the second length are equal to 4 or more and wherein the first and the second length differ from one another.

In embodiments, the length may correspond to the number of sequence units. In embodiments, the length may be up to 500 or less, e.g. up to 100 or less, preferably 50 or less, yet more preferably 20 or less. In embodiments, the first and the second length may be equal to 5 or more, preferably 6 or more. In embodiments, the characteristic biological subsequences may have a length between 4 and 20, preferably between 5 and 15, yet more preferably between 6 and 12.

In embodiments, the repository of fingerprint data strings may comprise at least 3 fingerprint data strings which differ in length from one another, preferably at least 4, yet more preferably at least 5, most preferably at least 6. Since the characteristic biological subsequences are not defined by their length, but by the number of possible sequence units which follow (or precede) it, a set of characteristic biological subsequences typically advantageously comprises subsequences of varying lengths. The repository of fingerprint data strings in the present invention differs from e.g. a collection of k-mers (as is known in the art) in that it comprises biological subsequences of varying lengths. Furthermore, a collection of k-mers typically comprises every permutation (i.e. every possible combination of sequence units) of fixed length k; this is not the case for the present repository of fingerprint data strings.

In embodiments, the fingerprint data strings may be protein fingerprint data strings, DNA fingerprint data strings or RNA fingerprint data strings or a combination thereof. In embodiments, the characteristic biological subsequence may be a characteristic protein subsequence, a characteristic DNA subsequence or a characteristic RNA subsequence. In embodiments, the repository of fingerprint data strings may comprise (e.g. consist of) protein fingerprint data strings, DNA fingerprint data strings, RNA fingerprint data strings or a combination of one or more of these. A characteristic protein subsequence can in embodiments be translated into a characteristic DNA or RNA subsequence, and vice versa. This translation can be based on the well-known DNA and RNA codon tables. Similarly, a protein fingerprint data string can be translated into a DNA or RNA fingerprint data string. In embodiments, a repository of DNA or RNA fingerprint data strings may comprise information on equivalent codons (i.e. codons which code for the same amino acid). This information on equivalent codons can be included in the fingerprint data string as such, or stored separately therefrom in the repository. In a particular embodiment, the fingerprint data strings may be in a format which is sequence-independent; meaning that the fingerprint data strings and surrounding systems and processes are such that they can be quickly compared to DNA, RNA and protein sequences. This can for example be achieved by having the methods which use the fingerprint data strings making the necessary translations on the fly. Such fingerprint data strings advantageously allow to formulate a single repository of data strings that is universally applicable across sequence types.

In embodiments, the repository of fingerprint data strings may further comprise additional data for at least one of the fingerprint data strings. In preferred embodiments, said data may be included in the fingerprint data string. In alternative embodiments, said data may be stored separately from the fingerprint data strings. In embodiments, the additional data may comprise one or more of combinatory data, structural data, relational data, positional data and directional data.

In embodiments, the combinatory data may be data related to one or more sequence units which can be consecutive to (e.g. which can realistically appear directly before or after, such as those combinations which are stable) the characteristic biological subsequence when said characteristic biological subsequence is present in a biological sequence. In embodiments, the combinatory data may comprise the number of possible sequence units, the possible sequence units as such, the likelihood (e.g. probability) for each sequence unit, etc.

In embodiments, the structural data may be structural information and/or spatial shape information embedded in the fingerprint data strings, such as data related to a secondary/tertiary/quaternary structure of the characteristic biological subsequence when said characteristic biological subsequence is present in a biopolymer. In embodiments, the structural data may comprise the number of possible structures, the possible structures as such, the likelihood (e.g. probability) for each structure, etc. In the case of multiple possible secondary/tertiary/quaternary structures for a given characteristic biological subsequence, the repository may in embodiments comprise a separate entry for each combination of the characteristic biological subsequence and an associated secondary/tertiary/tertiary structure. In alternative embodiments, the repository may comprise one entry comprising the characteristic biological subsequence and a plurality of its associated secondary/tertiary/quaternary structures. In embodiments, the secondary/tertiary/quaternary structure may be more relevant for proteins than for DNA and RNA-particularly the quaternary structure.

In embodiments, the relational data be data related to a relationship between the characteristic biological subsequence and one or more further characteristic biological subsequences. In embodiments, the relational data may comprise further characteristic biological subsequences which commonly appear in its vicinity, the likelihood for the further characteristic biological subsequence to appear in its vicinity, a particular significance (e.g. a biologically relevant meaning, such as a trait or a secondary/tertiary/quaternary structure) of these characteristic biological subsequences appearing close to one another, etc. In embodiments, the relationship may be expressed in the form of a path between two or more characteristic biological subsequences. In embodiments, the relationship may comprise an order of the characteristic biological subsequences and/or their interdistance. In embodiments, the additional data may also comprise metadata useful for building said paths.

In embodiments, the positional data may be data related to an interdistance with respect to the fingerprint data strings (e.g. between the characteristic biological sequences they represent).

In embodiments, the directional data may be data related to a direction (e.g. an inherent direction) of the fingerprint data strings (e.g. of the characteristic biological sequences they represent).

In some embodiments, the additional data may have been retrieved from a known data set; e.g. the secondary/tertiary/quaternary structure of several biological sequences is available in the art. In other embodiments, the additional data may have been may be extracted from a processed biological sequence as defined any embodiment of the fourth aspect or from a repository of processed biological sequences as defined in any embodiment of the sixth aspect. For example, after processing a biological sequence according to any embodiment of the third aspect (or building a repository of processed biological sequences according to any embodiment of the fifth aspect), relationships between the characteristic biological subsequences (e.g. paths) may be extracted and added to a repository of fingerprint data strings of the present aspect; this is schematically depicted in FIG 3 by the dashed arrows pointing from the processed biological sequence 210 and the repository of processed biological sequences 220 to the repository of fingerprint data strings 100.

In embodiments, the fingerprint data strings may be inherently directed. In embodiments, the fingerprint data strings may comprise a direction (i.e. may explicitly comprise the direction). Since HYFT^{™} fingerprints are defined based on actual fragments occurring in biopolymers or biopolymer fragments, the intrinsic physical, chemical and structural limitations that occur in nature for the occurring combinatory possibilities in biopolymers are inherently present in the HYFTs^{™}; where under 'inherently present' is understood that such information is (or at least can be) implicitly tied to the HYFT^{™}, even if it is not explicitly included as additional data in the repository. Therefore, since biological sequences as such normally have an inherent directionality (i.e. in accordance with the 5'-to-3' direction in DNA/RNA and the N-terminus to C-terminus in proteins), this same directionality is inherently present in the HYFTs^{™}. This link with actual fragments further defines restrictions in the maximum amount of biopolymer fragments that can follow after the last or prior the first character of a HYFT^{™}. The latter can also be explicitly expressed by a parameter (i.e. the combinatory number) that represents the total amount of next or previous possible combinations. This also results in the HYFT^{™} having an inherent (strict) direction.

In embodiments, the fingerprint data strings may comprise positional information. Characters in HYFTs^{™} as well as between HYFTs^{™} are interrelated on a syntactic level and therefore an interdistance between them or between different HYFTs^{™} can be defined. Such positions or interdistances belong to the positional information that can be inherently present in HYFTs^{™}.

In embodiments, the fingerprint data string also may comprise structural and/or spatial shape information. Also the possible structures and/or spatial shapes for certain HYFTs^{™} or combination of HYFTs^{™} is limited due to intrinsic physical, chemical and structural limitations. Such information is also inherently present in the HYFTs^{™} or sets of interrelated HYFTs^{™}.

In a second aspect, the present invention relates to a computer-implemented method for building and/or updating a repository of fingerprint data strings as defined in any embodiment of the first aspect, comprising: (a) identifying a characteristic biological subsequence in a biological sequence database, the characteristic biological subsequence having a combinatory number which is lower than the total number of different sequence units available thereto, the combinatory number of a biological subsequence being defined as the number of different sequence units that appear in the biological sequence database as a consecutive sequence unit of the biological subsequence; (b) optionally, translating the identified characteristic biological subsequence to one or more further characteristic biological subsequences; and (c) populating said repository with one or more fingerprint data strings representing the identified characteristic biological subsequence and/or the one or more further characteristic biological subsequences.

In a third aspect, the present invention relates to a computer-implemented method for processing a biological sequence, comprising: (a) retrieving one or more fingerprint data strings from the repository of fingerprint data strings as defined in any embodiment of the first aspect, (b) searching the biological sequence for occurrences of the characteristic biological subsequences represented by the one or more fingerprint data strings, and (c) constructing a processed biological sequence comprising for each occurrence in step b a fingerprint marker associated with the fingerprint data string which represents the occurring characteristic biological subsequence. FIG 3 schematically shows a sequence processing unit 310 which processes a biological sequence 200 using a repository of fingerprint data strings 100, thereby obtaining a processed biological sequence 210.

In some embodiments, the marker may be a reference string. Such a reference string may for example point towards the corresponding fingerprint data string in the repository. In other embodiments, the marker may be the fingerprint data string as such, or a portion thereof.

In embodiments, the biological sequence may comprise: (i) one or more first portions, each first portion corresponding to one of the characteristic biological subsequences represented by the one or more fingerprint data strings, and (ii) one or more second portions, each second portion not corresponding to any of the characteristic biological subsequences represented by the one or more fingerprint data strings. In embodiments, constructing the processed biological sequence in step c may comprise replacing at least one first portion by the corresponding marker. In embodiments, constructing the processed biological sequence in step c may further comprise adding positional information about said first portion to the processed biological sequence (e.g. appended to the marker). In embodiments, constructing the processed biological sequence in step c may comprise leaving at least one second portion unchanged, and/or replacing at least one second portion by an indication of the length of said second portion, and/or entirely removing at least one second portion. When leaving the second portions unchanged, the biological sequence is advantageously able to be processed in a completely lossless way.

In embodiments, the processed biological sequence can be formulated in a condensed format. For example, by replacing the characteristic biological subsequences (i.e. first portions) with reference strings and/or by replacing the second portions with either an indication of its length or entirely removing it, a processed biological sequence is obtained which requires less storage space than the original (i.e. unprocessed) biological sequence. Additional data compression can be achieved by making use of paths which can represent multiple fingerprints by their interrelation.

In embodiments, the one or more fingerprint data strings may be in a different biological format than the biological sequences (e.g. protein vs DNA vs RNA sequence information) and step b may further comprise translating or transcribing the characteristic biological subsequences prior to the searching.

In embodiments, the searching in step b may include searching for a partial match or an equivalent match (e.g. an equivalent codon, or a different amino acid resulting in the same secondary/tertiary/quaternary structure). In embodiments, the searching in step b may take into account a secondary/tertiary/quaternary structure of the characteristic biological subsequence. The secondary, tertiary and quaternary are typically more evolutionary conserved and often variation in the primary structure occur which do not change the function of the biopolymer, e.g. because the secondary/tertiary/quaternary structure of its active sites is substantially conserved. The secondary/tertiary/quaternary structure may therefore reveal relevant information about the biopolymer which would be lost when strictly searching for a fully matching primary structure.

In preferred embodiments, the searching for occurrences of the characteristic biological subsequences in step b may be performed in particular order. In embodiments, the order may be based on the length and the combinatory number of the characteristic biological subsequences. In embodiments, the search may be performed in order starting with the longest characteristics biological subsequences with the lowest combinatory number and ending with shortest characteristics biological subsequences with the highest combinatory number. In preferred embodiments, the order may be from longest to shortest characteristic biological subsequences and-for characteristic biological subsequences of the same length-from lowest to highest combinatory number. In other embodiments, the order of may be from lowest to highest combinatory number and-for characteristic biological subsequences with the same combinatory number-from longest to shortest characteristic biological subsequences. In embodiments, the order may further take into account additional data (e.g. to determine the order within a set of characteristic biological subsequences having the same length and same combinatory number), such as contextual data.

In embodiments, the method may comprise a further step d, after step c, of at least partially inferring a secondary/tertiary/quaternary structure of the processed biological subsequence based on the structural data as defined in embodiments of the first aspect. This at least partial elucidation of the secondary/tertiary/quaternary structure can help to assist and/or facilitate biological sequence design. In embodiments wherein a single primary structure of a characteristic biological subsequence is linked to a plurality of secondary or tertiary or quaternary structures, the secondary/tertiary/quaternary structure may be disambiguated based on the context in which the characteristic biological subsequence is found, such as the characteristic biological subsequences which it is surrounded by. The information needed for such disambiguation may, for example, be found in the repository of fingerprint data strings in the form of data (e.g. relational data) related to a relationship in terms secondary/tertiary/quaternary structure between the characteristic biological subsequence and one or more further characteristic biological subsequences, as defined in embodiments of the first aspect. For example, a particular first HYFT^{™} fingerprint may be known to adopt either a helix or turn configuration as a secondary structure, but to always adopt a helix configuration when a particular second HYFT^{™} fingerprint is present within a certain interdistance from said first HYFT^{™}. In such a case, the HYFT^{™} pattern of HYFT^{™} fingerprints-if observed-can be used to disambiguate the secondary structure of the first HYFT^{™}.

In embodiments wherein fingerprint data strings are inherently directed and comprise positional information, step c may comprises constructing the processed biological sequence as a directional graph, in embodiments, the directional graph may be a directional a-cyclical graph. It is to be noted that when reference is made to an a-cyclical graph, this does not imply that there are loops cannot occur, but it rather implies that the overall graph is not cyclical. The resulting graph representation for the reconstructed sequence as obtained in embodiments of the present invention may be referred to as a HYFT^{™} graph. Such a HYFT^{™} graph may allow for a universal genome graph representation.

In embodiments, constructing the processed biological sequence may comprise taking into account an interdistance between different fingerprint data strings, and/or may comprise taking into account a direction (e.g. an inherent direction) of the fingerprint data strings for constructing the directional graph.

In embodiments, constructing a processed biological sequence may comprise taking into account structural and /or spatial shape information embedded in the fingerprint data strings for constructing the directional graph, and/or may comprise taking into account syntactical information embedded in the fingerprint data strings.

In embodiments, the searching in step b may take into account any of positional information, interdistance information between different elements of the characteristic biological sequence, a secondary and/or tertiary and/or quaternary structure of the characteristic biological subsequence and/or a structural variation of the characteristic biological subsequence.

By way of illustration, embodiments of the present invention not being limited thereto, an example of how a certain sequence can be searched is shown below. The method comprises in a first step identifying a HYFT^{™} being present in the sequence to be searched. The method then further comprises querying the reference database by searching all sequences in the reference database that also contain that HYFT^{™}. The different sequences found are then sorted, e.g. sorted by length and the location of the HYFT^{™} in the sequence is identified. Furthermore aligning is performed. In some embodiments, aligning may be performed using Navarro-Levenshtein matching. A more detailed description of the Navarro-Levenshtein matching can for example be found in Navarro, Theoretical Computer Science 237 (2000) 455-463. Aligning may be performed with a directed graph, e.g. a directed a-cyclical graph. The latter may be a universal genome reference graph, although embodiments are not limited thereto. The aligning may include identification of variants for a certain sequence. In order to perform the above steps, the sequence may be further processed, whereby for example dead ends and loops may be removed.

In a fourth aspect, the present invention relates to a processed biological sequence, obtainable by the computer-implemented method according to any embodiment of the third aspect. A processed biological sequence 210 is schematically depicted in FIG 3.

In a fifth aspect, the present invention relates to a computer-implemented method for building and/or updating a repository of processed biological sequences, comprising populating said repository with processed biological sequences as defined in any embodiment of the fourth aspect. FIG 3 schematically shows a repository building unit 320 storing a processed biological sequence 210 into a repository of processed biological sequences 220.

In a sixth aspect, the present invention relates to a repository of processed biological sequences, obtainable by the computer-implemented method according to any embodiment of the fifth aspect. A repository of 220 is schematically depicted in FIG 3.

In embodiments, the repository of processed biological sequences may be combined with the repository of fingerprint data strings.

In embodiments, the repository may be a database. In some embodiments, the repository of processed biological sequences may be an indexed repository. The repository may, for example, be indexed based on the fingerprint markers (corresponding to the characteristic biological subsequences) present in each processed biological sequence. In other embodiments, the repository may be a graph repository.

In a seventh aspect, the present invention relates to a computer-implemented method for comparing a first biological sequence to a second biological sequence, comprising: (a) processing the first biological sequence by the computer-implemented method according to any embodiment of the third aspect to obtain a first processed biological sequence, or retrieving the first processed biological sequence from a repository of processed biological sequences as defined in any embodiment of the sixth aspect, (b) processing the second biological sequence by the computer-implemented method according to any embodiment of the third aspect to obtain a second processed biological sequence, or retrieving the second processed biological sequence from a repository of processed biological sequences as defined in any embodiment of the sixth aspect, and (c) comparing at least the fingerprint markers in the first processed biological sequence with the fingerprint markers in the second processed biological sequence. FIG 4 schematically shows a comparison unit 330 comparing at least a first biological sequence 211 and a second biological sequence 212 to output results 400.

By using characteristic biological subsequences according to embodiments of the present invention (through the fingerprint markers in the processed biological sequences), the problem of comparing sequences is advantageously reformulated from an NP-complete or NP-hard problem to a polynomial-time problem. Indeed, identifying the fingerprints in a sequence and subsequently comparing sequences based on these fingerprints, which can be considered as a lexical approach, is computationally much simpler than the currently used algorithms (which e.g. compare full sequences based on a sliding windows approach). The comparison can therefore be performed markedly faster and furthermore scales well with increasing complexity (e.g. increasing length of or number of biological sequences), even while requiring less computation power and storage space.

In embodiments, step c may comprise identifying whether one or more characteristic biological subsequences (represented by the fingerprint markers) in the first processed biological sequence correspond (e.g. match) with one or more characteristic biological subsequences (represented by the fingerprint markers) in the second processed biological sequence. In embodiments, step c may comprise identifying whether the corresponding characteristic biological subsequences appear in the same order in the first processed biological sequence as in the second processed biological sequence. In embodiments, step c may comprise identifying whether one or more pairs of characteristic biological subsequences in the first processed biological sequence and one or more corresponding pairs of characteristic biological subsequences in the second processed biological sequence have a same or similar (e.g. differing by less than 1000 sequence units, e.g. less than 100 sequence units, preferably less than 50 sequence units, yet more preferably less than 20 sequence units, most preferably less than 10 sequence units) interdistance.

In embodiments, step c may further comprise comparing one or more second portions of the first processed biological sequence with one or more second portions in the second processed biological sequence. In embodiments, comparing one or more second portions may comprise comparing corresponding second portions (i.e. a second portion appearing in between a neighbouring pair of characteristic biological subsequences in the first processed biological sequence and a second portion appearing in between a corresponding neighbouring pair of characteristic biological subsequences in the first processed biological sequence).

In embodiments, step c may further comprise calculating a measure representing a degree of similarity (e.g. a Levenshtein distance) between the first and the second biological sequence. In embodiments, the degree of similarity may be calculated based on a plurality of variables, such as combining a measure of syntactic similarity with a measure of structural similarity.

In embodiments, the method may be used in a sequence similarity search, by comparing a query sequence with one or more other biological sequences (e.g. corresponding to a sequence database that is to be searched, for example in the form of a repository of processed biological sequences). In embodiments, a degree of similarity may be calculated for each of the other biological sequences. In embodiments, the method may comprise a further step of ranking the biological sequences (e.g. by decreasing degree of similarity). In embodiments, the method may comprise filtering the biological sequences. Filtering may be performed before and/or after step c. For example, filtering may be performed by selecting for comparison only those biological sequences from the database which fit a certain criterion, such as based on the organism or group of organisms which they derive from (e.g. plants, animals, humans, microorganisms, etc.), whether a secondary/tertiary/quaternary structure is known, their length, etc. Alternatively, filtering may be performed after the comparison has been performed, based on the same criteria or based on the calculated degree of similarity (e.g. only those sequences may be selected which surpass a certain threshold of similarity). In contrast to sequence similarity searching in the prior art, where an alignment step is typically required and a measure of similarity is then established therefrom, alignment is not strictly necessary for similarity searching. Indeed, similar sequences can already be found by simply searching for sequences with the same fingerprints (optionally also taking into account their order and their interdistance), without alignment; this in turn allows to further speed up the search. The above notwithstanding, alignment (cf. infra) is also computationally simplified, so that it may be chosen to do an alignment anyway, even if not strictly required.

The method of this aspect thus allows determining (and optionally measuring) the similarity between a first and a second biological sequence. Such a comparison is also a cornerstone in other methods, such as those for aligning and assembling (cf. infra).

In embodiments, the method may be for aligning a first biological sequence to a second biological sequence. In embodiments, step c may further comprise aligning the fingerprint markers in the first processed biological sequence with the fingerprint markers in the second processed biological sequence. FIG 4 schematically shows output results 400 from comparison unit 330 (which is in this case better referred to as 'alignment unit 330') in which biological sequences are aligned by their fingerprint markers.

Alignment is thus also simplified in embodiments, since a good alignment can already be obtained by simply aligning the fingerprints. Once more, this significantly reduces the computational complexity of the problem. Furthermore, in the prior art methods, such as those based on progressive alignment, there is a build-up of alignment errors, as misalignment for one of the earlier sequences typically propagate and cause additional misalignments in the later sequences. Conversely, since it is each time the same discrete set of fingerprint markers which are aligned (or at least attempted to) within one (multiple) alignment, there is no such propagation of errors.

In embodiments, the method may further comprise subsequently aligning corresponding second portions. Aligning the second portions may, for example, be performed using one of the alignment methods known in the prior art. Indeed, since the 'skeleton' of the alignment is already provided by aligning the fingerprint markers, only the alignment in between these markers is left to be fleshed out. Since each of these second portions is typically relatively short compared to the total biological sequence length, the known methods can typically perform such an alignment relatively quickly and efficiently.

In embodiments, the method may be for performing a multiple sequence alignment (i.e. the method may comprise aligning three or more biological sequences). In embodiments, the method may comprise aligning fingerprint markers in a third (or fourth, etc.) processed biological sequence with fingerprint markers in the first and/or second processed biological sequences. This is schematically depicted in FIG 4 in which alignment unit 330 may also compare and align an arbitrary number of further processed biological sequences 213-216.

In embodiments, the method may be used in variant calling. In the case of sequence alignment between two biological sequences, the variant calling may identify variants (e.g. mutations) between a query sequence and a reference sequence. In the case of a multiple sequence alignment, the variant calling may identify the possible variations (which may include determining their frequency of occurrence) in a set of related sequences; optionally with respect to a reference sequence. Identifying variants may furthermore be performed on the basis of the primary structure, but may also take account of the secondary/tertiary/quaternary structure. Identifying variants thus may be performed based on the primary structure, based on secondary/tertiary/quaternary structure, but also based on every possible interrelation of distances correlated to the HYFT^{™} in the sequence, or to distance information with respect to a next or previous HYFT^{™}. Identifying variants may also be based on variations of the codon table, thus allowing to gather immediate info about DNA, RNA and amino acid variations in the same variant analysis.

In embodiments, the method may be for performing a sequence assembly. In embodiments, the method may comprise: (a) providing a first biological sequence, the first biological sequence being a biological sequence of a first biopolymer fragment, (b) providing a second biological sequence, the second biological sequence being either a biological sequence of a second biopolymer fragment or being a reference biological sequence, (c) aligning the first biological sequence to the second biological sequence as described above, and (d) merging the first biological sequence with the second biological sequence to obtain an assembled biological sequence. FIG 5 schematically shows a sequence assembling unit 340 outputting assembled biological sequence 510, by first aligning (by their fingerprint markers) and subsequently merging an arbitrary number of biological sequences 500 (comprising of at least a first biological sequence 501 and second biological sequence 502).

In embodiments, the method steps a to d may be repeated so as to align and merge an arbitrary number of biopolymer fragments.

In order to facilitate sequencing, longer biopolymers can be fragmented, since the individual fragments are sequenced faster and more easily (e.g. they can be sequenced in parallel); as is known in the art. Sequence assembly is then typically used to align and merge fragment sequences to reconstruct the original sequence; this may also be referred to as 'read mapping', where 'reads' from a fragment sequence are 'mapped' to a second biopolymer sequence. Depending on the type of sequence assembly that is being performed, e.g. a de-novo assembly vs. a mapping assembly, the second biopolymer sequence may be selected to be a second biopolymer fragment or a reference sequence, as appropriate. Herein, a de-novo assembly is an assembly from scratch, without using a template (e.g. a backbone sequence). Conversely, a mapping assembly is an assembly by mapping one or more biopolymer fragment sequences to an existing backbone sequence (e.g. a reference sequence), which is typically similar (but not necessarily identical) to the to-be-reconstructed sequence. A reference sequence may for example be based on (part of) a complete genome or transcriptome, or may be have been obtained from an earlier de-novo assembly.

In embodiments, the method may comprise a further step e, after step d, of aligning the assembled biological sequence to the second biological sequence as described above. This additional alignment may be used to perform variant calling of the assembled biological sequence with respect to the second biological sequence (e.g. the reference sequence).

In an eighth aspect, the present invention relates to a storage device comprising a repository of fingerprint data strings according to any embodiment of the first aspect and/or a repository of processed biological sequences according to any embodiment of the sixth aspect.

It may furthermore relate to a processing system comprising such a storage device and further comprising a processor adapted for obtaining fingerprint data strings from the storage device and/or for storing fingerprint data strings to the storage device and/or searching in fingerprint data strings in the storage device.

In a ninth aspect, the present invention relates to a data processing system adapted to (e.g. comprising means therefor) carry out the computer-implemented method according to any embodiment of the second, third, fifth or seventh aspect.

The system may typically take on a different form depending on the method(s) it is meant to carry out. In embodiments, the system may be or comprise a sequence processing unit, a repository building unit, a comparison unit, an alignment unit, a variant calling unit or a sequence assembling unit. In embodiments, a generic data processing means (e.g. a personal computer or a smartphone) or a distributed computing environment (e.g. cloud-based system) can be configured to perform one or more of these functions. The distributed computing environment may, for example, comprise a server device and a networked client device. Herein, the server device may perform the bulk of one or more methods, including storing the repository of fingerprint data strings and the repository of processed biological sequences. On the other hand, the networked client device may communicate instructions (e.g. input, such as a query sequence, and settings, such as search preferences) with the server device and may receive the method output.

In a tenth aspect, the present invention relates to a computer program (product) comprising instructions which, when the program is executed by a computer (system), cause the computer to carry out a computer-implemented method according to any embodiment of the second, third, fifth or seventh aspect.

The present invention also relates to a computer program product comprising instructions which, when the program is executed by a computer system, cause the computer system for carrying out obtaining, searching or storing fingerprint data strings respectively from, in or to the repository of fingerprint data strings.

In an eleventh aspect, the present invention relates to a computer-readable medium comprising instructions which, when executed by a computer (system), cause the computer to carry out a computer-implemented method according to any embodiment of the second, third, fifth or seventh aspect.

In a twelfth aspect, the present invention relates to a use of a repository of fingerprint data strings as defined in any embodiment the first aspect, for one or more selected from: processing a biological sequence, building a repository of processed biological sequences, comparing a first biological sequence to a second biological sequence, aligning a first biological sequence to a second biological sequence, performing a multiple sequence alignment, performing a sequence similarity search and performing a variant calling.

In a thirteenth aspect, the present invention relates to a use of a processed biological sequence as defined in any embodiment of the fourth aspect or a repository of processed biological sequences as defined in any embodiment as defined in any embodiment of the sixth aspect, for one or more selected from: comparing a first biological sequence to a second biological sequence, aligning a first biological sequence to a second biological sequence, performing a multiple sequence alignment, performing a sequence similarity search and performing a variant calling.

In embodiments, any feature of any embodiment of any of the above aspects may independently be as correspondingly described for any embodiment of any of the other aspects.

A detailed description of several embodiments will now be shown. It is clear that other embodiments can be configured according to the knowledge of the person skilled in the art without departing from the true technical teaching of such embodiments, the embodiments being limited only by the terms of the appended claims.

### Example 1: Processing of the Protein Data Bank in accordance with the present invention

### Example 1a: Analysis of the Protein Data Bank with respect to the HYFT^{™} fingerprints found therein

In order to illustrate the pervasive presence of HYFT^{™} fingerprints in biological sequence databases, the Protein Data Bank (PDB) was taken as an example of a large, commonly available biological sequence database and was processed-in accordance with the present invention-using a repository of fingerprint data strings obtained as described above. The results were analysed with respect to various indicators and a selection thereof is presented below.

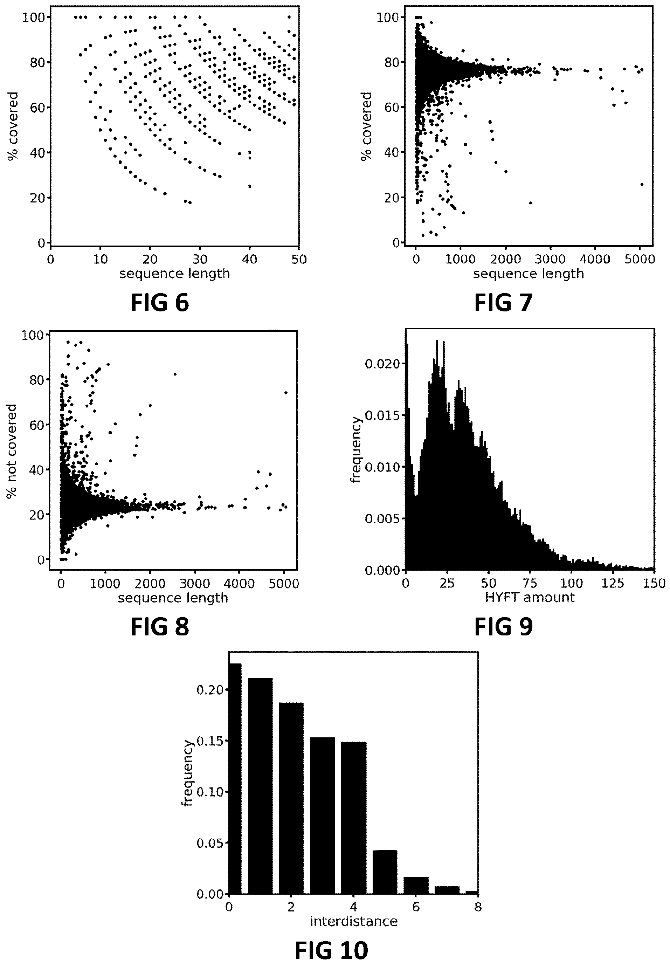

FIG 6 and FIG 7 show the HYFT^{™} coverage ratios (in %) for processed protein sequences up to length 50 and up to lengths over 5000, respectively. Here, the coverage ratio is the part of the total sequence length of which the sequence units were attributed to a HYFT^{™} fingerprint. In other words, the coverage ratio is the combined length of the one or more first portions divided by the total sequence length.

The inverse statistic, i.e. the part of the total sequence length not covered by a HYFT^{™} fingerprint (or the combined length of the one or more second portions divided by the total sequence length), is shown in FIG 8 for the case of lengths up to over 5000.

Tied in to the above, FIG 9 gives an overview of the number of HYFTs^{™} retrieved per processed sequence in the form of a frequency distribution.

Remarkably, these charts show that at least one HYFT^{™} fingerprint was found in every processed biological sequence; indeed, not a single PDB sequence was not covered by one or more HYFTs^{™}. Moreover, long sequences are widely covered by HYFT^{™} patterns, with the coverage spread generally thinning as the sequence length increases. On average, a coverage rate of close to 80% is achieved.

Typical interdistances that were observed are shown in FIG 10, whichdepicts the frequency distribution of the length of the second portions appearing before and after a HYFT^{™} fingerprint.

Overall the above results support that virtually every protein sequence (and by extension DNA and/or RNA sequence) can be rewritten as a string of one or more HYFTs^{™} (i.e. HYFT^{™} patterns) on the basis of a repository of HYFT^{™} fingerprint data strings in accordance with the present invention. Moreover, because of the good coverage rate that is generally achieved, the processed sequences still retain the essential characteristics of their unprocessed counterparts; especially when not solely the identified HYFTs^{™} are retained, but this is expanded with additional data (cf. supra) such as the interdistances (i.e. the length of the second portions) before, between and after the identified HYFTs^{™}. A highly performant indexing based on HYFT^{™} patterns can be achieved-with near perfect retrieval rates.

### Example 1b: Effect of the matching strategy employed

Since different strategies can be employed when processing a biological sequence in accordance with the present invention, the difference between two different approaches was investigated. In a first approach, the biological sequences in the PDB database were searched for all occurrences of HYFT^{™} fingerprints, including overlapping HYFTs^{™}, so that the order in which the HYFT^{™} fingerprints becomes immaterial. In a second approach, the biological sequenced in the PDB database were searched using a more strict fashion, wherein the searching is performed in order of from longest to shortest HYFT^{™} fingerprints and-within the same length-from lowest to highest combinatory number and wherein no overlap of HYFTs^{™} is allowed (i.e. wherein a portion found to be corresponding to a HYFT^{™} is from then on excluded in search for further HYFTs^{™}). The goal of the second approach beingto identify the fewest number of HYFTs^{™} to describe a processed biological sequence while still ensuring good coverage of the sequence, by disallowing overlap and by favouring stricter HYFTs^{™} (i.e. longer length with lower combination number) over less strict HYFTs^{™} (i.e. shorter length with higher combination number).

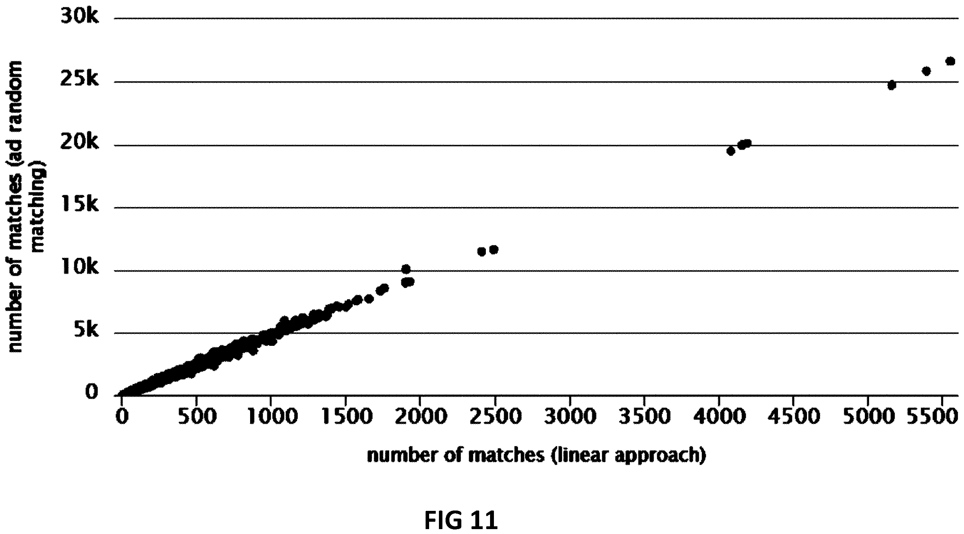

The number of different matches found per biological sequence are plotted against one another in FIG 11. As can be observed, a generally linear relationship is found with indeed roughly about 5 times fewer matches for the stricter second approach than for the first approach. These fewer matches amount to an increase in processing time-both to identify the HYFT^{™} fingerprints and to subsequently use the processed sequences in further methods-and storage space needed; while nevertheless sufficiently fully characterizing the whole sequence. As such, it is believed that the second approach strikes an optimal balance and is generally preferred.

The above notwithstanding, note however that the number and nature of the matches found using the first approach is lower and better than a comparable k-mer approach. As such, although the second approach may be generally preferred over the first, the first approach nevertheless remains advantageous over the known-art methods.

### Example 2: Comparison between a sequence search as known in the prior art and one in accordance with an embodiment of the present description

### Example 2a: Using a short search string

Two separate searches were performed based on the search string "AVFPSIVGRPRHQGVMVGMGQKDSY". This corresponds to a relatively short protein sequence with a length of 25 sequence units, which could for example be a protein fragment in protein sequencing.

The first search was performed using BLAST (Basic Local Alignment Search Tool); more particularly 'Protein BLAST' (available at the url: https://blast.ncbi.nlm.nih.gov/Blast.cgi?PROGRAM=blastp&PAGE TYPE=BlastSearch&L INK LOC=blasthome). The following search parameters were used: Database = Protein Data Bank proteins (pdb); Algorithm = blastp (protein-protein BLAST); Max target sequences = 1000; Short queries = Automatically adjust parameters for short input sequences; Expect threshold = 20000; Word size = 2; Matrix = PAM30; Compositional adjustment = No adjustment. BLAST required over 30 seconds for this search, after which 604 search results were returned.

On the other hand, based on the principles of the present embodiment, it was determined that "IVGRPRHQGVM" is a characteristic biological subsequence (i.e. a 'HYFT^{™} fingerprint') comprised in the above short protein sequence. As such, the second search was performed in a repository of processed biological sequences based on the search string "IVGRPRHQGVM". This repository was based on the same protein database as used in BLAST (i.e. Protein Data Bank; PDB), which had been previously processed using a repository of fingerprint data strings (cf. supra); i.e. characteristic biological subsequences represented by the fingerprint data strings were identified and marked in a set of publicly available biological sequences. This search returned 661 results. In contrast to BLAST, the time frame needed in this case was only 196 milliseconds. As such, even for such a relatively short sequence, it was observed that the present method was able to reduce the required time by a factor of over 150 compared to the known-art method.

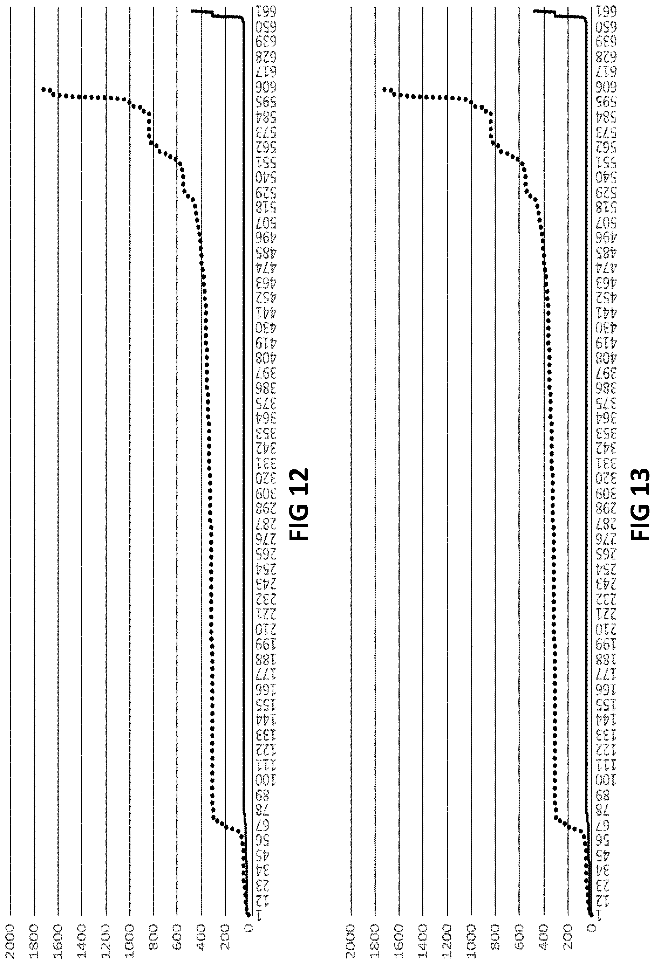

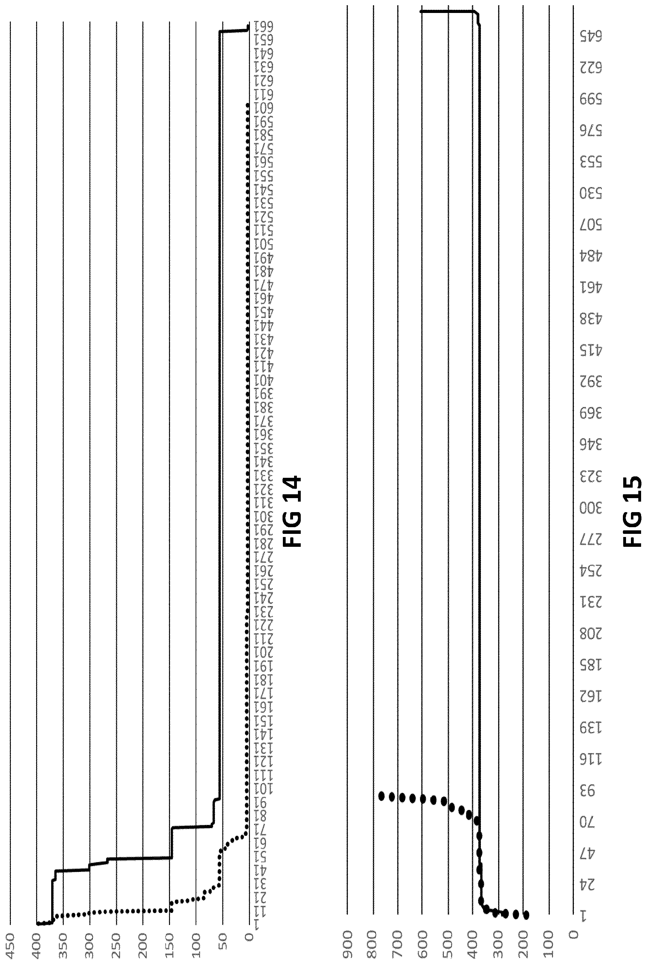

We now refer to FIG 12, FIG 13 and FIG 14, showing the results of both of these searches (BLAST = dotted line; present method = solid line) in terms of their total length (FIG 12), their Levenshtein distance (FIG 13) and longest common substring (FIG 14). For each graph, the search results are shown ordered from low to high with respect to the plotted parameter (i.e. total length, Levenshtein distance or longest common substring). Furthermore, one of the search result, namely the protein sequence 5NW4_V (i.e. the first result listed by BLAST), was selected as a reference with respect to which the Levenshtein distance and the longest common substring were calculated. As can be observed in these figures, the present method yielded, across the full range of search results, a smaller variation in total length (characterized by a relative plateau spanning over a significant portion of the results), a considerably lower Levenshtein distance and a considerably larger longest common substring; compared to the BLAST results. The combination of these suggests that the method of the present embodiment was able to identify results which are more relevant for the performed search.

### Example 2b: Using a longer protein as the search string

The previous example was repeated, but this time a complete protein sequence, 3MN5_A (with a length of 359 sequence units), was searched.

The first search, using BLAST, returned 88 search results.

On the other hand, based on the principles of the present embodiment, it was determined that six characteristic biological subsequences (i.e. 'HYFT^{™} fingerprints') could be found in the sequence 3MN5_A; these were denoted as:

```
       +4641474444415052415646_1,+495647525052485147564d_1,
       +4949544e5744444d454b49_1, +494d464554464e5650414d_1,
       +494b454b4c435956414c44_1 and +49474d4553414749484554_1,
```

where e.g. '49474d4553414749484554' corresponds to the respective subsequence in hexadecimal format. As such, the second search was performed, in the same repository of processed biological sequences as in the previous example, to find those protein sequences which comprise the same six characteristic biological subsequences in the same order. This search returned 661 results.

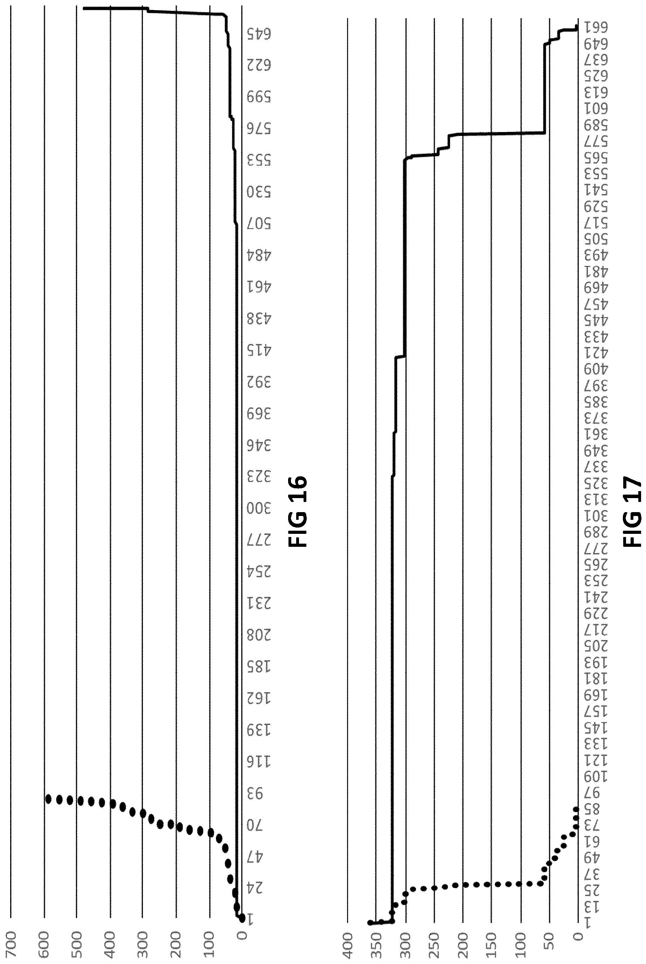

We now refer to FIG 15, FIG 16 and FIG 17, showing the results of both of these searches (BLAST = dotted line; present method = solid line) in terms of their total length (FIG 15), their Levenshtein distance (FIG 16) and longest common substring (FIG 17). For each graph, the search results are shown ordered from low to high with respect to the plotted parameter (i.e. total length, Levenshtein distance or longest common substring). In this case, the Levenshtein distance and the longest common substring were calculated with respect to the original query sequence 3MN5_A. As can be observed in these figures, the characteristics of the search results for both methods are relatively comparable at the extremes. However, the present method yielded in the intermediate range a plateau of results with little variation in total length, a low Levenshtein distance and a fairly high longest common substring. The combination of these suggests that the method of the present embodiment was able to identify a larger number of relevant results.

It is to be understood that although preferred embodiments, specific constructions and configurations, as well as materials, have been discussed herein for devices according to the present embodiments, various changes or modifications in form and detail may be made without departing from the scope and technical teachings of this description. For example, any formulas given above are merely representative of procedures that may be used. Functionality may be added or deleted from the block diagrams and operations may be interchanged among functional blocks. Steps may be added or deleted to methods described within the scope of the present embodiments.

## Claims

1. A repository of fingerprint data strings (100) for a biological sequence database, each fingerprint data string representing a characteristic biological subsequence made up of sequence units, the sequence units being amino acids when the characteristic biological subsequence relates to a protein and codons when the characteristic biological subsequence relates to DNA or RNA,
each characteristic biological subsequence having in the biological sequence database a combinatory number which is 15 or fewer, the combinatory number of a biological subsequence being defined as the number of different sequence units that appear in the biological sequence database as a consecutive sequence unit of the biological subsequence,
the characteristic biological subsequences having varying lengths of between 4 and 20 sequence units.

2. The repository of fingerprint data strings (100) according to claim 1, further comprising for at least one of the fingerprint data strings:
- data related to one or more sequence units which can be consecutive to the characteristic biological subsequence when said characteristic biological subsequence is present in a biological sequence; and/or
- data related to a secondary and/or tertiary and/or quaternary structure of the characteristic biological subsequence when said characteristic biological subsequence is present in a biopolymer; and/or
- data related to a relationship between the characteristic biological subsequence and one or more further characteristic biological subsequences.

3. A computer-implemented method for building and/or updating a repository of fingerprint data strings (100) as defined in any of claims 1 to 2, comprising:
a. identifying a characteristic biological subsequence in a biological sequence database, the characteristic biological subsequence having a combinatory number which is 15 or fewer, the combinatory number of a biological subsequence being defined as the number of different sequence units that appear in the biological sequence database as a consecutive sequence unit of the biological subsequence,
the characteristic biological subsequences having varying lengths of between 4 and 20 sequence units;
b. optionally, translating the identified characteristic biological subsequence to one or more further characteristic biological subsequences; and
c. populating said repository (100) with one or more fingerprint data strings representing the identified characteristic biological subsequence and/or the one or more further characteristic biological subsequences.

4. A storage device comprising a repository of fingerprint data strings (100) according to claim 1 or 2.

5. A data processing system (310, 320, 330) adapted to carry out the computer-implemented method according to claim 3.

6. A computer program or computer-readable medium comprising instructions which, when executed by a computer, cause the computer to carry out the computer-implemented method according to claim 3.
